# EUROPEAN PATENT SPECIFICATION

(11) **EP 4 090 272 B1**
(45) Date of publication and mention of the grant of the patent: **05.08.2026**
(21) Application number: 20909472.1
(22) Date of filing: 24.12.2020
(51) Int. Cl.: A61B 17/80, A61B 17/68, A61B 17/70, A61B 17/82, A61B 17/00

(54) **STERNUM COVER PLATE USED FOR STERNUM FIXATION HAVING AN ADJUSTABLE HEIGHT**
BRUSTBEINABDECKPLATTE ZUR BRUSTBEINFIXIERUNG MIT EINSTELLBARER HÖHE
PLAQUE DE RECOUVREMENT DE STERNUM À HAUTEUR RÉGLABLE UTILISÉE POUR FIXER LE STERNUM

(30) Priority: 31.12.2019 TR 201923144
(43) Date of publication of application: 23.11.2022
(73) Proprietor: Çulhalik, Selin, Atakum/Samsun (TR)
(72) Inventor: ÇULHALIK, Serdar, Atakum/Samsun (TR)
(86) International application number: PCT/TR2020/051375
(87) International publication number: WO 2021/137817

(56) References cited:
- CN-A- 108 042 191
- CN-A- 109 998 614
- CN-A- 110 123 428
- CN-U- 204 033 454
- CN-U- 208 371 871
- US-A1- 2005 267 475
- US-A1- 2011 313 474

## Description

### Technical Field

The invention is related to a sternum cover plate that can be applied to have a size that is compliant with the anatomical structure, which is placed into the body with the purpose of sealing the bone during its recovery process after it has been cut following a sternotomy opening surgery, which is a cardiovascular surgery. Said plate/implant can be absorbed within the body after the desired treatment (bone recovery, integration) is provided.

### Prior Art

Nowadays, it is reported that more than 1 million cardiac surgeries have been made around the world, together with an increase in cardiovascular population. Most of these operations are carried out with sternum dissection or sternotomy.

Sternotomy is a surgical intervention performed by means of opening the sternum from the center of chest cavity. This method is usually preferred in cardiac surgeries.

In these types of operations, the sternum is opened and the organs located at the lower section can be reached with the purpose of surgical intervention.

Following a surgical intervention, the sternum that has been opened should be closed back and it has to be joined afterwards for providing bone bonding.

In a similar manner, in the case of a fracture of the sternum due to different reasons, the sternum should be joined and bone bonding should be waited.

However, due to the position of the bone and its features, healing of sternum fractures is problematic. Due to constant breathing motion, the chest heaves and rests, causing the healing period of the sternum fractures to be prolonged.

In this long time period, it is of upmost importance to hold the fractured sternum together. In the prior art, there are several different apparatuses used in order to keep the sternum together.

As an example of these apparatuses, screwed connection apparatuses can be given. These apparatuses are fixed by means of a screw and they aim to keep the sternum together, and thereby enable bone bonding.

These screwed connection apparatuses are called sternum cover plate fixators.

The application number CN108742813 mentions a similar sternum plate. The sternum plate described comprises a male claw fixing piece, a female claw fixing piece and a locking piece. A male holding claw has been provided at the end of the male claw fixing piece and a nail fixation has been provided at the end of the female holding claw. Thanks to this structure, the plate is removably connected.

It can be understood that the structure subject to application number CN108742813 is unidimentionally formed.

Since there are different sternum dimensions and the geometry of the area of application varies, these implants are manufactured in sets having different dimensions, and the correct size of implant is applied for the patient.

As is known, implants have high prices because of their raw materials. Therefore, a considerable inventory expense is put forth in order to store and manufacture several different implants for different patients as well as for different areas of incision.

In order to remedy this situation, it is preferred to manufacture the implant having the correct size for the patient, said implant is produced after taking the measurements of the patient, however; this method is far from being advantageous since it will prolong supply time.

In order to eliminate these problems, implants with changeable dimensions are created. Thereby, the number of implants which have different dimensions to be stocked is decreased.

Within the scope of this solution, implants having several height ranges are prepared and these implants can be used by changing their measurements within their range.

Patent application number WO2019028969 mentions a similar sternum cover plate fixator. It is understood that the fixator mentioned in the description is used with the purpose of closing the sternum. It is also understood that the height of the fixator mentioned in the application can be changed.

The fixator mentioned in the document has a structure in which the height can be changed due to the expandable adjustment of the distance between the inner claw and the outer claw.

As a result, it will be possible to store only two different dimensions for the use of the related fixator. On the other hand, the smaller size can be adjusted at suitable dimensions for the surgical interventions applied for children.

However, the fixator mentioned in the document still does not have the suitable structure to be operated under particular dimensions.

CN110123428 (A) describes a rapidly-disassembled sternum closure fixer designed to enhance the efficiency and safety of sternal closure procedure. The described implementation is a screwed sternum cover plate designed for sealing the sternum during recovery from fractures and cuts. It consists of an outer claw and an inner claw that interlock to form an adjustable plate, allowing it to fit securely on the sternum. The design includes screw mounting spaces created by the interlocking claws, as well as hooks at both ends of the plate for enhanced stability. Additionally, an apparatus connection member enables the locking of the outer and inner claws after they are coupled, ensuring a secure closure during the healing process.

### Problems Aimed to be Solved by the Invention

The present invention is defined in claim 1 while preferred embodiments are set forth in the dependent claims.

The purpose of the invention is to create a sternum cover plate which enables opening with different dimensions.

The purpose of the invention is also to provide a sternum cover plate which enables the creation of a sternum cover plate that enables opening with very small dimensions. Thereby, the use of separate sternum cover plates for different anatomical bone structures in many different dimensions is prevented.

As a result, the obligation of having access to different products that vary for different anatomical structures and that need to be and determined during surgery is eliminated. This reduces the high inventory expenses of sternum plates.

On the other hand, the sternum cover plate of the invention provides an advantage due to its hook structure which is made suitable for the anatomical structure by being elongated to suit thicker sternum bones.

### Description of the Figures

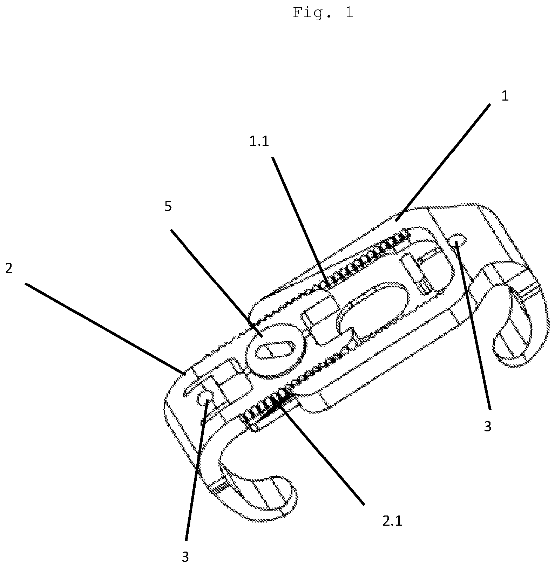

- Figure 1.: Top perspective view of the screwed sternum cover plate,
- Figure 2.: Side view of the screwed sternum cover plate,
- Figure 3.: Top perspective view of the outer claw,
- Figure 4.: Top view of the inner claw,

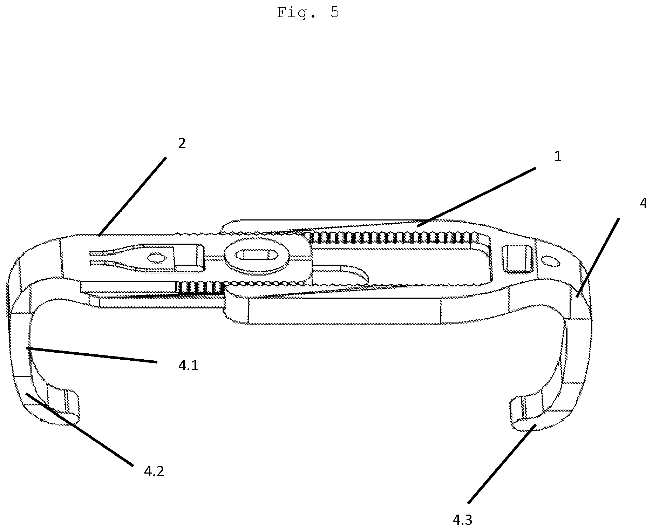

- Figure 5.: Top perspective view of the medium sized screwed sternum cover plate,

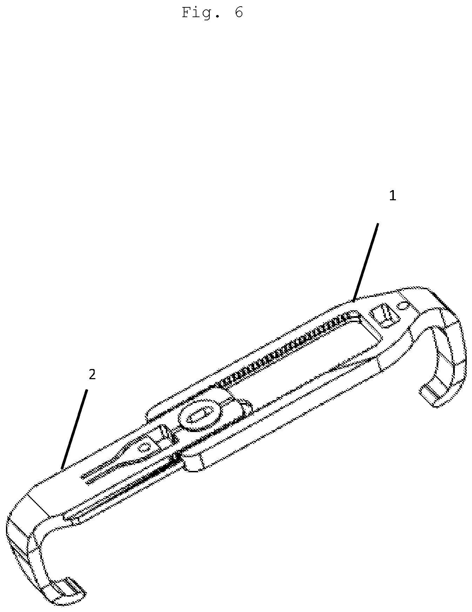

- Figure 6.: Top perspective view of the large sized screwed sternum cover plate,

### Description of the References in the Figures

- 1.: Outer claw
1.1. Outer claw guide rail
1.2. Indentation
- 2.: Inner claw
2.1. Inner claw guide rail
- 3.: Screw mounting space
- 4: . Hook
4.1. Straight piece
4.2. Spring piece
4.3. Thread piece
- 5.: Apparatus connection member
- 6.: Protrusion
- 7.: Hole
- 8.: . Housing

### Description of the Invention

The invention is related to a screwed sternum cover plate for treating the sternum fractures and cuts that is used during the recovery period of the sternum in order to seal it.

Said screwed sternum cover plate is basically composed of;
- an outer claw (1) and inner claw (2) that can be closed by interlocking with each other and/or whose size can be changed thereby to form the plate sitting on the sternum by being joined together,
- screw mounting spaces (3) which are formed on the plate by the interlocking of the outer claw (1) and inner claw (2),
- 2 hooks (4) that are formed at the ends of the plate that is formed by the interlocking of said outer claw (1) and inner claw (2),
- an apparatus connection member (5) that enables the connection of the used apparatus in order for the outer claw (1) and inner claw (2) to lock after they are coupled to each other.

The outer claw (1) can be coupled to the inner claw (2) over the outer claw guide rail (1.1) that is provided around its outer edges. Thereby, the height of the sternum plate can be changed by the movement of the inner claw (2) within the outer claw (1).

In a similar way, the movement of the inner claw (2) within the outer claw (1) will enable the plate to be opened and closed during its installation onto the sternum.

The ability of the inner claw (2) to be mounted within the outer claw (1) is provided by means of the overlapping of the outer claw guide rail (1.1) and inner claw guide rail (2.1).

With the purpose of providing the movement of the two pieces within each other only by means of force, the outer claw guide rail (1.1) and inner claw guide rail (2.1) comprise threads in their structure, which are compliant with each other.

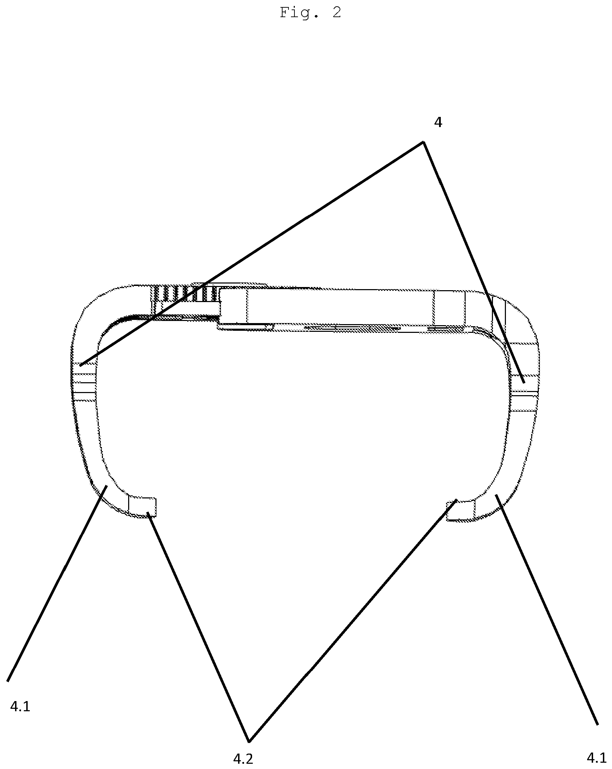

According to Figure 2, the hook was given a special form in order for the hook (4) to better grip the lower section of the sternum.

According to this form, the hook (4) is composed of a straight piece (4.1) which extends in a straight direction from the connection twist of the inner claw (2) formed at the edges of the plate that is formed by the outer claw (1), a spring piece (4.2) having a spring form at the continuation of the straight piece (4.1) and a thread piece (4.3) which is formed as an inwards thread at the continuation of the spring piece (4.2).

According to the preferred embodiment of the invention, the hook (4) formed by the above mentioned pieces (4.1, 4.2, 4.3) is uniform.

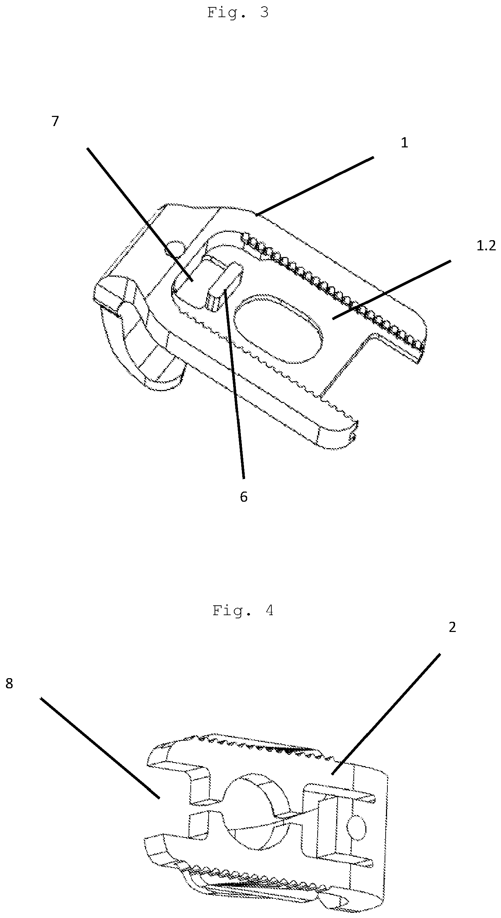

According to Figure 3, an indentation (1.2) formed at the mid-section of the outer claw (1) has been provided. Within this indentation (1.2), at least one protrusion (6) has been provided.

As a result, the inner claw (2) is provided with a housing (8) preferably formed at the end of its body. Since the housing (8) aims to include the protrusion (6) within itself, its structure and dimensions are compliant with the protrusion (6).

According to Figure 3, the protrusion (6) is provided with at least one hole (7) on its side.

Within the preferred embodiment of the invention, the hole (7) is positioned at the distal end of the indentation (1.2). In this case, the hole (7) is provided with the protrusion (6) at its end towards the center.

Since the indentation (1.2) formed within the outer claw (1) aims to receive the inner claw (2) within itself, the sizes and dimensions of the indentation (1.2) and the inner claw (2) are compliant such as to enable this layout.

According to the preferred embodiment of the invention, the inner claw (2), outer claw (1) and hooks (3) are manufactured from biodegradable materials.

As a result, the implant of the invention will be able to be used without being removed after it is placed in the body.

Therefore the implant of the invention can be manufactured from materials that can be dissolved within the time predicted for the bonding of the sternum.

## Claims

1. A screwed sternum cover plate that is used to seal the sternum during the recovery period in order to treat sternum fractures and cuts comprising:
an outer claw (1) and inner claw (2) which can be closed by interlocking with each other the plate is formed by the interlocking of the outer claw (1) and inner claw (2) and screw mounting spaces (3) which are formed on this plate,
two hooks (4) that are formed at the ends of the plate that is formed by the interlocking of said outer claw (1) and inner claw (2), an apparatus connection member (5) that enables the connection of the used apparatus in order for the outer claw (1) and inner claw (2) to lock after they are coupled to each other,
-**characterized in that** each hook (4) comprises a straight piece (4.1) which extends straight from the connection twist of the inner claw (2) and of the outer claw (1) formed at the edges of the plate, a spring piece (4.2) having a spring form at the continuation of each straight piece (4.1) and a thread piece (4.3) which is formed as an inward thread at the continuation of each spring piece (4.2).

2. The screwed sternum cover plate according to Claim 1, **characterized in that** it comprises a hook (4) composed of a straight piece (4.1) which extends straight from the connection twist of the inner claw (2) formed at the edges of the plate that is formed by the outer claw (1), a spring piece (4.2) having a spring form at the continuation of the straight piece (4.1) and a thread piece (4.3) which is formed as an inward thread at the continuation of the spring piece (4.2).

3. The screwed sternum cover plate according to Claim 1, **characterized in that** it comprises:
• an outer claw (1) provided with an indentation (1.2) at its mid-section and at least one protrusion (6) within the indentation (1.2),
• an inner claw (2) which is provided with a housing (8) whose structure and dimensions are compliant with the protrusion (6).

4. The screwed sternum cover plate according to Claim 3, **characterized in that** it comprises an inner claw (2) provided with a housing (8) that is formed at its end.

5. The screwed sternum cover plate according to Claim 3, **characterized in that** it comprises a protrusion (6) provided with at least one hole (7) on its side.

6. The screwed sternum cover plate according to Claim 5, **characterized in that** it comprises a hole (7) positioned at the distal end of the indentation (1.2) and which is provided with the protrusion (6) at its end towards the center.

7. The screwed sternum cover plate according to Claim 1, **characterized in that** it comprises an outer claw guide rail (1.1) and an inner claw guide rail (2.1) provided with thread structures that are compliant with each other.

8. The screwed sternum cover plate according to any of the preceding Claims, **characterized in that** it is manufactured from biodegradable materials.

## Patentansprüche

1. Eine verschraubte Brustbeinabdeckungsplatte zur Abdichtung des Brustbeins während der Heilungsphase nach Brustbeinfrakturen und -schnitten, bestehend aus: einer äußeren Klaue (1) und einer inneren Klaue (2), die durch Ineinandergreifen verschlossen werden können; die Platte wird durch das Ineinandergreifen der äußeren Klaue (1) und der inneren Klaue (2) sowie durch auf dieser Platte ausgebildete Schraubaufnahmen (3) gebildet; zwei
Haken (4), die an den Enden der durch das Ineinandergreifen der äußeren Klaue (1) und der inneren Klaue (2) gebildeten Platte ausgebildet sind; ein Geräteanschlusselement (5), das den Anschluss des verwendeten Geräts ermöglicht, sodass die äußere Klaue (1) und die innere Klaue (2) nach dem Verbinden miteinander verriegelt werden; **dadurch gekennzeichnet, dass** jeder Haken (4)
ein gerades Stück umfasst. (4.1) welches sich gerade von der Verbindungsdrehung der inneren Klaue (2) und der äußeren Klaue (1) erstreckt, die an den Plattenrändern ausgebildet sind, ein Federstück (4.2) mit einer Federform an der Fortsetzung jedes geraden Stücks (4.1) und ein Gewindestück (4.3), das als Innengewinde an der Fortsetzung jedes Federstücks (4.2) ausgebildet ist.

2. Die verschraubte Brustbeinabdeckungsplatte nach Anspruch 1, **dadurch gekennzeichnet, dass** sie einen Haken (4) umfasst, der aus einem geraden Stück (4.1) besteht, welches sich gerade von der Verbindungsdrehung der inneren Klaue (2) erstreckt, die an den Plattenrändern ausgebildet ist und von der äußeren Klaue (1) gebildet wird, einem Federstück (4.2) mit einer Federform an der Fortsetzung des geraden Stücks (4.1) und einem Gewindestück (4.3), das als Innengewinde an der Fortsetzung des Federstücks (4.2) ausgebildet ist.

3. Die verschraubte Brustbeinabdeckung nach Anspruch 1, **dadurch gekennzeichnet, dass** sie Folgendes umfasst:
• eine äußere Klaue (1) mit einer Vertiefung (1.2) in ihrer Mitte und mindestens einem Vorsprung (6) innerhalb der Vertiefung (1.2),
• eine innere Klaue (2) mit einem Gehäuse (8), dessen Struktur und Abmessungen mit dem Vorsprung (6) kompatibel sind.

4. Die verschraubte Brustbeinabdeckung nach Anspruch 3, **dadurch gekennzeichnet, dass** sie eine innere Klaue (2) mit einem an ihrem Ende ausgebildeten Gehäuse (8) umfasst.

5. Die verschraubte Brustbeinabdeckung nach Anspruch 3, **dadurch gekennzeichnet, dass** sie einen Vorsprung (6) mit mindestens einer seitlichen Öffnung (7) umfasst.

6. Die verschraubte Brustbeinabdeckung nach Anspruch 5, **dadurch gekennzeichnet, dass** sie eine Öffnung (7) am distalen Ende der Vertiefung (1.2) umfasst, die mit dem Vorsprung (6) an ihrem mittig gerichteten Ende versehen ist.

7. Die verschraubte Brustbeinabdeckung nach Anspruch 1, **dadurch gekennzeichnet, dass** sie eine äußere Klauenführungsschiene (1.1) und eine innere Klauenführungsschiene (2.1) mit zueinander passenden Gewindestrukturen aufweist.

8. Die verschraubte Brustbeinabdeckung nach einem der vorhergehenden Ansprüche, **dadurch gekennzeichnet, dass** sie aus biologisch abbaubaren Materialien hergestellt ist.

## Revendications

1. Une plaque de recouvrement sternal vissée, utilisée pour sceller le sternum pendant la période de convalescence afin de traiter les fractures et les plaies du sternum, comprenant : une griffe externe (1) et une griffe interne (2) qui peuvent être fermées par emboîtement ; la plaque est formée par l'emboîtement de la griffe externe (1) et de la griffe interne (2) et des espaces de montage de vis (3) formés sur cette plaque ; deux crochets (4) formés aux extrémités de la plaque formée par l'emboîtement desdites griffes externe (1) et interne (2) ; un élément de connexion (5) permettant la connexion de l'appareil utilisé afin que les griffes externe (1) et interne (2) se verrouillent après leur accouplement ; **caractérisée en ce que** chaque crochet (4) comprend une pièce droite (4.1) qui s'étend en ligne droite depuis la torsion de liaison de la griffe intérieure (2) et de la griffe extérieure (1) formée aux bords de la plaque, une pièce à ressort (4.2) présentant une forme de ressort dans le prolongement de chaque pièce droite (4.1) et une pièce filetée (4.3) constituée d'un filetage intérieur dans le prolongement de chaque pièce à ressort (4.2).

2. La plaque de recouvrement de sternum vissée selon la revendication 1, **caractérisée en ce qu'**elle comprend un crochet (4) composé d'une pièce droite (4.1) qui s'étend en ligne droite depuis la torsion de liaison de la griffe intérieure (2) formée aux bords de la plaque par la griffe extérieure (1), d'une pièce à ressort (4.2) présentant une forme de ressort dans le prolongement de la pièce droite (4.1) et d'une pièce filetée (4.3) constituée d'un filetage intérieur dans le prolongement de la pièce à ressort (4.2).

3. La plaque de recouvrement sternal vissée selon la revendication 1, **caractérisée en ce qu'**elle comprend :
• une griffe extérieure (1) munie d'une échancrure (1.2) en son milieu et d'au moins une saillie (6) à l'intérieur de cette échancrure (1.2),
• une griffe intérieure (2) munie d'un logement (8) dont la structure et les dimensions sont compatibles avec la saillie (6).

4. La plaque de recouvrement sternal vissée selon la revendication 3, **caractérisée en ce qu'**elle comprend une griffe intérieure (2) munie d'un logement (8) formé à son extrémité.

5. La plaque de recouvrement sternal vissée selon la revendication 3, **caractérisée en ce qu'**elle comprend une saillie (6) munie d'au moins un orifice (7) sur son côté.

6. La plaque de recouvrement sternal vissée selon la revendication 5, **caractérisée en ce qu'**elle comprend un trou (7) situé à l'extrémité distale de l'indentation (1.2) et muni d'une protubérance (6) à son extrémité, orientée vers le centre.

7. La plaque de recouvrement sternal vissée selon la revendication 1, **caractérisée en ce qu'**elle comprend un rail de guidage extérieur (1.1) et un rail de guidage intérieur (2.1) munis de filetages compatibles entre eux.

8. La plaque de recouvrement sternal vissée selon l'une quelconque des revendications précédentes, **caractérisée en ce qu'**elle est fabriquée à partir de matériaux biodégradables.
